# FASCICULE DE BREVET EUROPEEN

(11) **EP 2 651 491 B1**
(45) Date de publication et mention de la délivrance du brevet: **12.09.2018**
(21) Numéro de dépôt: 11817340.0
(22) Date de dépôt: 14.12.2011
(51) Int. Cl.: A61M 39/22, A61M 1/02

(54) **DISPOSITIF DESTINE A ROMPRE AU MOINS UN ELEMENT DE FERMETURE DISPOSE A L'INTERIEUR D'UN TUBE SOUPLE**
VORRICHTUNG ZUM BRECHEN VON MINDESTENS EINEM VERSCHLUSSELEMENT INNERHALB EINES SCHLAUCHES
DEVICE INTENDED TO BREAK AT LEAST ONE CLOSURE ELEMENT LOCATED INSIDE A FLEXIBLE TUBE

(30) Priorité: 14.12.2010 FR 1004885
(43) Date de publication de la demande: 23.10.2013
(73) Titulaire: Maco Pharma, 59420 Mouvaux (FR)
(72) Inventeur: DEVERRE, Frédéric, 34090 Montpellier (FR); BONTINCK, Pierre, Eloi, 59800 Lille (FR); CHAVATTE, Arnaud, 62330 Isbergues (FR); SCHROEDER, Toni, 63303 Dreieich (DE)
(74) Mandataire: Sayettat, Julien Christian
(86) Numéro de dépôt international: PCT/FR2011/052992
(87) Numéro de publication internationale: WO 2012/080664

(56) Documents cités:
- WO-A2-2004/058046
- US-A1- 2010 132 512

## Description

L'invention concerne un dispositif destiné à rompre un élément de fermeture disposé à l'intérieur d'un tube souple, ainsi qu'un appareil comprenant un tel dispositif et un procédé pour rompre un élément de fermeture à l'aide d'un tel dispositif.

Elle s'applique typiquement dans les domaines de la transfusion et de la perfusion à la rupture des éléments de fermeture placés à l'intérieur d'un tube souple d'un système à poches. Ces éléments de fermeture sont généralement désignés comme des ouvre-circuits.

Les ouvre-circuits sont des dispositifs de vanne logés à l'intérieur d'un tube souple, empêchant l'écoulement de fluide jusqu'à la rupture manuelle de l'ouvre-circuit au moment où un produit sanguin ou autre solution doit passer à l'intérieur de ce tube. Un exemple de tels ouvre-circuits est décrit dans le document WO-93/17734.

Certains centres de transfusion traitent près de 8 000 dons de sang par jour. La rupture de ces ouvre-circuits, à l'occasion de la collecte du sang et de la séparation des composants sanguins, est une étape répétitive et contraignante engendrant au niveau du personnel médical des troubles musculo-squelettiques. Les pathologies les plus répandues sont les tendinites, le syndrome du canal carpien ou les ténosynovites.

Le document WO-2004/058046 propose d'automatiser l'ouverture des ouvre-circuits en fournissant un crochet rétractable intégré à un appareil de traitement de sang. La casse de l'ouvre-circuit est réalisée par un mouvement linéaire ou rotatif faisant plier l'ouvre-circuit d'un seul côté. Cette manipulation ne permet pas de s'assurer de la rupture totale de l'ouvre-circuit.

Le document WO-2010/065396 propose un dispositif portable d'ouverture des ouvre-circuits cassables comprenant deux crochets, mobiles en sens opposé l'un par rapport à l'autre, et permettant un mouvement de va et vient pour casser l'ouvre-circuit. Ce type de dispositif présente l'inconvénient de faire bouger la poche lors de la casse de l'ouvre-circuit. Lorsque la poche contient différents composants sanguins séparés par centrifugation, la casse de l'ouvre-circuit avec un tel dispositif est susceptible d'entraîner le re-mélange des composants sanguins, ce qui n'est pas souhaitable.

L'invention vise à résoudre ces différents problèmes en proposant notamment un dispositif, qui est particulièrement simple à réaliser et à utiliser. Ce dispositif permet également de diminuer le temps de main d'oeuvre, d'assurer une casse efficace et reproductible de tout type d'ouvre-circuits, et de diminuer les opérations sur les poches centrifugées pouvant altérer la séparation. Enfin, l'intégration de ce dispositif dans un appareil de traitement d'un fluide biologique permet de minimiser les risques d'erreurs liés aux manipulations des systèmes à poches.

A cet effet, et selon un premier aspect, l'invention concerne un dispositif destiné à rompre au moins un élément de fermeture disposé à l'intérieur d'un tube souple, ledit élément de fermeture comportant une première partie et une deuxième partie séparées par une zone de fragilité, ladite zone de fragilité pouvant être rompue pour permettre l'écoulement de fluide à l'intérieur dudit tube souple, ledit dispositif comprenant un ensemble de réception dudit tube souple, ledit ensemble comprenant un élément fixe pourvu d'un premier logement destiné à recevoir une première portion dudit tube souple, et un élément mobile pourvu d'un deuxième logement destiné à recevoir une deuxième portion dudit tube souple, les deux logements étant alignés suivant un axe médian définissant une position neutre de l'élément mobile, ledit dispositif comprenant un organe d'entraînement en déplacement de l'élément mobile de part et d'autre de sa position neutre, de sorte à pouvoir provoquer la rupture de la zone de fragilité de l'élément de fermeture lorsque le tube souple est placé dans l'ensemble de réception.

Selon un deuxième aspect, l'invention concerne un procédé pour rompre un élément de fermeture à l'aide d'un dispositif selon le premier aspect, comprenant les étapes de :
- placer le tube souple comportant l'élément de fermeture dans l'ensemble de réception dudit dispositif avec la zone de fragilité disposée entre l'élément fixe et l'élément mobile,
- actionner l'organe pour entraîner en déplacement l'élément mobile de part et d'autre de sa position neutre de sorte à provoquer la rupture de la zone de fragilité de l'élément de fermeture.

La figure 1 représente une vue schématique d'un système à poches utilisé pour la collecte et la séparation des composants sanguins.
La figure 2A représente une vue schématique d'un élément de fermeture dans un tube souple dans un état non rompu.
Les figures 2B et 2C représentent une vue schématique de la rupture de l'élément de fermeture de la figure 2A par pliage dans un sens et dans l'autre de l'élément de fermeture.
La figure 3A représente une vue schématique d'un élément mobile et d'un élément fixe d'un ensemble de réception du dispositif de l'invention selon une première réalisation.
Les figures 3B à 3D représentent de façon schématique les étapes de la rupture d'un élément de fermeture logé dans un tube souple et disposé sur l'ensemble de réception de la figure 3A, par déplacement linéaire de l'élément mobile.
La figure 4A représente une vue schématique d'un élément mobile et d'un élément fixe d'un ensemble de réception du dispositif de l'invention selon une autre réalisation.
Les figures 4B à 4D représentent de façon schématique les étapes de la rupture d'un élément de fermeture logé dans un tube souple et disposé sur l'ensemble de réception de la figure 4A, par rotation de l'élément mobile.
La figure 5 représente de façon schématique une autre réalisation d'un ensemble de réception selon l'invention dans lequel l'élément fixe comprend une pince.
La figure 6 est une vue partielle en perspective d'un dispositif selon une réalisation de l'invention sur lequel est disposé une poche.
La figure 7 est une vue agrandie d'une partie du dispositif et de la poche de la figure 6.
La figure 8 est une vue partielle et en perspective de l'organe d'entraînement et du système d'engrenage du dispositif de la figure 6.
La figure 9 est une vue partielle et de face d'un dispositif selon une autre réalisation de l'invention destiné à rompre simultanément deux éléments de fermeture, une poche étant disposé sur le dispositif.
La figure 10 est une vue partielle et arrière de l'organe d'entraînement et du système d'engrenage du dispositif de la figure 9.

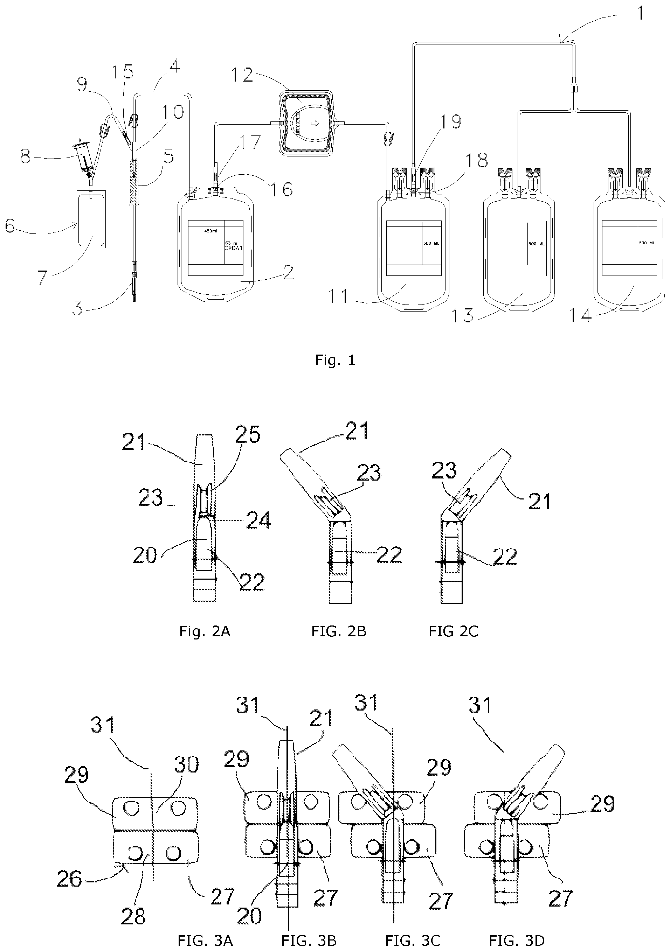

La figure 1 représente un système à poches 1 utilisé pour la collecte du sang et la séparation des composants sanguins en transfusion. Ce système à poches 1 est typiquement composé d'une poche 2 de collecte du sang connectée à une aiguille de prélèvement 3 par l'intermédiaire d'un premier tube souple 4. Un protecteur d'aiguille 5 est placé sur le premier tube souple 4 et est destiné à recouvrir l'aiguille 3 à la fin du prélèvement sanguin.

Un ensemble d'échantillonnage 6 comprenant une poche d'échantillonnage 7 et un dispositif de transfert 8 vers un tube sous vide (non représenté) est connecté au premier tube souple 4 par l'intermédiaire d'un deuxième tube souple 9. Les premier et deuxième tubes souples sont connectés entre eux par l'intermédiaire d'un connecteur en Y 10. Cet ensemble d'échantillonnage 6 est utilisé pour échantillonner le sang prélevé en vue d'effectuer des analyses.

La poche de collecte 2 est en outre reliée par l'intermédiaire d'un troisième tube souple à une poche primaire 11. Sur la figure 1, un filtre 12 à déleucocyter le sang total est disposé sur ce troisième tube souple. La poche primaire 11 est destinée à recueillir le sang filtré au travers du filtre 12 à déleucocyter. La poche primaire 11 est en communication fluidique avec deux poches secondaires 13,14 destinées à recevoir respectivement le plasma et le concentré de globules rouges obtenus après centrifugation de la poche primaire 11 contenant le sang total filtré.

La poche de collecte 2 comprend un anticoagulant de type ACD (acide citrate dextrose) ou CPD (citrate phosphate dextrose). Pour éviter que l'anticoagulant ne pénètre dans l'ensemble d'échantillonnage et fausse les analyses, un premier élément de fermeture 15 est disposé au niveau du connecteur en Y 10. De même, un des orifices de sortie 16 de la poche de collecte est pourvu d'un deuxième élément de fermeture 17 pour éviter que l'anticoagulant ne se déplace dans le reste du système à poches 1, notamment lors de la production de ces systèmes, pendant l'étape de stérilisation.

Sur la figure 1, un troisième élément de fermeture 18 est disposé au niveau d'un orifice 19 de la poche primaire 11 pour, lors de la centrifugation de la poche primaire 11, éviter d'envoyer le sang non séparé dans les poches secondaires 13,14.

Il existe plusieurs types d'éléments de fermeture. Généralement et comme représenté sur les figures 2A à 2C, un élément de fermeture 20 est disposé à l'intérieur d'un tube souple 21. L'élément de fermeture comportant une première partie 22, désignée embase, et une deuxième partie 23, désignée plume. Ces deux parties 22,23 sont séparées par une zone de fragilité 24, ladite zone de fragilité pouvant être rompue pour permettre l'écoulement de fluide à l'intérieur dudit tube souple 21.

Plus en détail, la première partie 22 de l'élément de fermeture 20 est formée d'un cylindre creux dont le diamètre extérieur correspond sensiblement au diamètre intérieur dudit tube souple 21 dans lequel l'élément de fermeture est inséré. Ainsi, le diamètre extérieur de cette première partie 22 est sensiblement égal ou légèrement supérieur au diamètre intérieur du tube souple 21 empêchant ainsi le passage du fluide sur les côtés de l'embase 22 de l'élément de fermeture.

La deuxième partie 23 de l'élément de fermeture 20 est formée d'un cylindre ou d'un cône plein. Dans le tube souple 21, le fluide est capable de circuler autour de cette deuxième partie 23. Sur les figures 2A à 2C, la deuxième partie comprend des ailettes 25, facilitant le passage de fluide autour de la plume 23, après rupture de la zone de fragilité 24.

Pour permettre l'écoulement du fluide, l'utilisateur manipule le tube souple 21 de l'extérieur pour faire plier la deuxième partie 23 de l'élément de fermeture 20 afin de réaliser la rupture de la zone de fragilité 24 et séparer les deux parties 22,23 de l'élément de fermeture (figures 2B et 2C). Une fois séparé, le fluide peut s'écouler à l'intérieur de la première partie 22 de l'élément de fermeture.

Selon un premier aspect, l'invention concerne un dispositif destiné à rompre au moins un élément de fermeture disposé à l'intérieur d'un tube souple.

Comme décrit ci-dessus, l'élément de fermeture comporte une première partie et une deuxième partie séparées par une zone de fragilité, ladite zone de fragilité pouvant être rompue pour permettre l'écoulement de fluide à l'intérieur dudit tube souple.

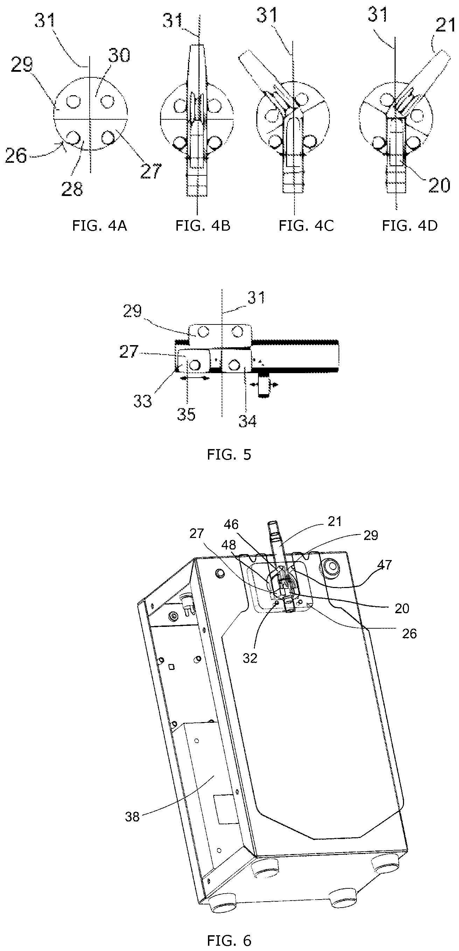

Comme représenté sur les figures 3A à 3D et 4A à 4D, le dispositif comprend un ensemble de réception 26 du tube souple 21 à l'intérieur duquel est disposé l'élément de fermeture 20. L'ensemble 26 comprend un élément fixe 27 pourvu d'un premier logement 28 destiné à recevoir une première portion dudit tube souple, et un élément mobile 29 pourvu d'un deuxième logement 30 destiné à recevoir une deuxième portion dudit tube souple, les deux logements 28,30 étant initialement alignés suivant un axe médian 31. Dans cet état initial, l'élément mobile 29 est dit dans une position neutre dans laquelle le tube souple 21 peut être placé dans l'ensemble de réception 26.

Les deux éléments fixe et mobile 27,29 sont disposés l'un au dessus de l'autre. Les logements 28,30 sont arrangés initialement pour maintenir l'élément de fermeture 20 dans son tube 21 dans un état non rompu.

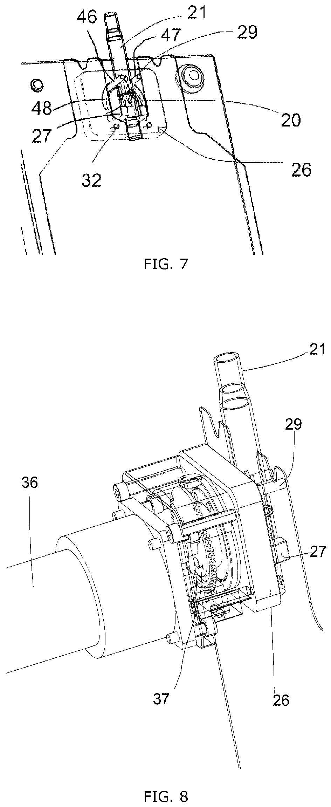

Selon une réalisation, au moins un des logements 28 est formé à l'intérieur d'une gorge en forme de U comme représenté sur les figures 6 et 7 pour le logement 28 de l'élément fixe 27.

Pour maintenir fermement l'élément fixe 27 lors du déplacement de l'élément mobile 29 d'un côté et de l'autre de l'axe médian 31, la longueur du logement 28 de l'élément fixe 27 est sensiblement égale à la longueur de l'élément fixe 27.

Le logement 30 de l'élément mobile 29 se compose de deux tiges parallèles 46,47 séparées d'une distance sensiblement équivalente au diamètre extérieur du tube souple 21.

Pour permettre le déplacement de l'élément mobile 29 par rapport à l'élément fixe 27, l'ensemble de réception comprend une cavité 48 correspondant à la forme du mouvement de l'élément mobile. Sur les figures 6 et 7, le mouvement de l'élément mobile est un mouvement de rotation et la cavité possède une forme en arc de cercle.

Généralement, les poches de transfusion comprennent des oeillets de suspension. Des tiges 32 peuvent être prévues sur l'ensemble de réception 26 pour recevoir lesdits oeillets de suspension. Ces tiges sont disposées sur l'ensemble de réception 26 de façon à ce que le ou les éléments de fermeture 20 se logent directement dans les logements 28,30 prévus. Cette configuration facilite la mise en place des éléments de fermeture 20 dans le dispositif.

Selon une autre réalisation, au moins l'un des logements 28 comprend une pince destinée à serrer une portion du tube souple 21.

Par élément fixe, on entend un élément qui ne se déplace pas lors de la rupture de la zone de fragilité de l'élément de fermeture. L'élément fixe peut être mobile avant ou après les étapes de rupture.

Par élément mobile, on entend un élément qui se déplace lors de la rupture de la zone de fragilité de l'élément de fermeture. Lors du déplacement de l'élément mobile pour réaliser la casse de l'élément de fermeture, l'élément fixe reste immobile.

Par exemple sur la figure 5, l'élément fixe 27 comprend une pince 33 formée de deux tiges 34,35 dont l'une 34 est fixe et l'autre 35 est mobile en translation linéaire pour ajuster l'écartement des deux tiges 34,35 au diamètre du tube souple 21.

Les logements 28,30 de l'élément fixe 27 et de l'élément mobile 29 sont alignés suivant l'axe médian 31. Cet axe médian correspond sensiblement à l'axe du tube souple 21 lorsqu'il est disposé dans les deux logements 28,30. Dans cette position initiale avec les deux logements alignés, l'élément mobile 29 est dit dans une position neutre. L'élément mobile 29 étant dans cette position initiale neutre, l'élément de fermeture 20 est dans son état non rompu comme représenté sur les figures 3B et 4B.

Le dispositif comprend un organe 36 d'entraînement en déplacement de l'élément mobile 29 de part et d'autre de sa position neutre, de sorte à pouvoir provoquer la rupture de la zone de fragilité 24 de l'élément de fermeture 20 lorsque le tube souple 21 est placé dans l'ensemble de réception 26. Ainsi, l'élément mobile 29 est déplaçable d'un côté et de l'autre de l'axe médian 31, entre une position décalée d'un côté dudit axe et une position décalée de l'autre côté dudit axe.

Lors de l'utilisation du dispositif de rupture, l'élément mobile 29 effectue un mouvement de va et vient au-delà de sa position neutre, permettant de séparer efficacement la première et la deuxième partie 22,23 de l'élément de fermeture 20.

Selon une première réalisation, illustrée sur les figures 3A à 3D, l'élément mobile 29 est entraîné en déplacement selon un mouvement linéaire. L'élément mobile se déplace alors perpendiculairement à l'axe médian 31, vers la droite et vers la gauche, dans un mouvement de va et vient en repassant par la position neutre.

Selon une deuxième réalisation plus avantageuse illustrée sur les figures 4A à 4D, l'élément mobile 29 est entraîné en déplacement selon un mouvement de rotation. Dans ce cas, l'axe de rotation est perpendiculaire à l'axe médian 31.

Pour minimiser l'effort nécessaire à la casse de la zone de fragilité 24, l'axe de rotation de l'élément mobile 29 passe par l'axe médian 31.

De façon avantageuse, pour assurer la rupture de la zone de fragilité 24 de l'élément de fermeture 20, le mouvement de rotation de l'élément mobile 29 est un mouvement d'au moins 30°, de préférence d'au moins 45°, de part et d'autre de l'axe médian 31.

Le mouvement de rotation réduit le risque d'arrachement ou de déformation du tube souple par rapport au mouvement de translation rectiligne de la première réalisation.

Sur les figures 8 à 10, l'organe d'entraînement 36 est par exemple un moteur dont l'arbre de rotation est rendu solidaire de l'élément mobile 29. Un système d'engrenage 37 comprenant au moins deux roues dentées permettent l'entraînement dudit élément mobile 29. Le type de mouvement, linéaire ou angulaire, est déterminé par la configuration du système d'engrenage 37.

Le moteur est alimenté électriquement, par des batteries ou directement par le courant électrique via une prise de courant.

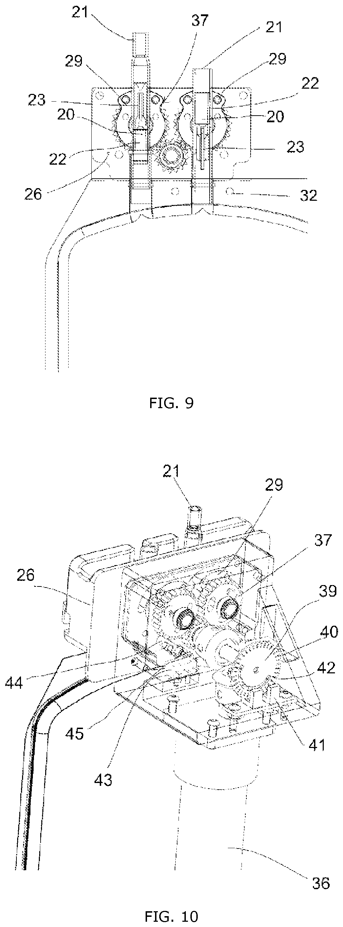

Selon une variante illustrée sur les figures 9 et 10, le dispositif est destiné à rompre deux éléments de fermeture 20, simultanément ou non.

Pour ce faire, le dispositif peut comprendre un autre ensemble de réception d'un autre tube souple. Cet ensemble de réception est similaire à celui qui vient d'être décrit, avec un autre élément fixe et un autre élément mobile, monté sur l'organe 36 qui est agencé pour entraîner en déplacement les organes mobiles 29 suivant un mouvement de rotation ou linéaire (non représenté). En variante, chaque organe mobile 29 peut être équipé de son propre organe d'entraînement en déplacement.

Toutefois, lorsque le dispositif est destiné à rompre simultanément les deux éléments de fermeture montés dans un tube souple, respectivement, il est avantageux de n'utiliser qu'un seul organe d'entraînement. Comme montré sur les figures 9 et 10, les éléments mobiles 29 sont alors montés sur ledit organe d'entraînement 36, de sorte à provoquer la rupture simultanée de la zone de fragilité de deux éléments de fermeture 20. Cette réalisation est plus compacte et s'applique notamment à l'ouverture simultanée de deux éléments de fermeture montés dans des tubes souples voisins formant des orifices d'accès d'une poche souple.

Dans ce cas, il est possible que les éléments de fermeture 20 soient disposés dans un sens opposé : pour l'un des éléments de fermeture, son embase 22 est montée plus près de la poche et pour l'autre élément, la plume 23 est montée plus près de la poche (figure 9).

En utilisation normale, l'élément fixe 27 est destiné à recevoir l'embase 22 de l'élément de fermeture 20 et l'élément mobile 29, la plume 23. Cependant, le dispositif fonctionne également lorsque l'élément de fermeture 20 est placé dans un sens inverse. Dans ce cas, c'est la plume 23 qui vient se loger dans l'élément fixe 27 et l'embase 22 dans l'élément mobile 29.

Il existe sur le marché plusieurs types d'éléments de fermeture ayant chacun une configuration et des dimensions propres. Il est envisagé de fabriquer des ensembles de réception appropriés pour un ou plusieurs types d'éléments de fermeture sous forme d'un panneau amovible destiné à être inséré dans le système d'engrenage.

Une unité de commande 38 permet de contrôler le fonctionnement du dispositif de rupture et notamment l'actionnement de l'organe 36 d'entraînement en déplacement de l'élément mobile 29. L'unité de commande 38 est par exemple un microprocesseur monté sur une carte mère.

En particulier, l'unité de commande et de contrôle fait fonctionner et vérifie le fonctionnement de l'organe d'entraînement 36 du dispositif.

Comme représenté sur la figure 10, l'organe d'entraînement 36, comme l'élément mobile 29, est solidaire d'une roue pleine 39 comportant une pluralité de cavités périphériques 40. En variante, une roue dentée est utilisée. La rotation de cette roue pleine 39 permet de contrôler le mouvement de l'élément mobile 29. Pour faire se déplacer l'élément mobile 29, l'organe d'entraînement tourne d'abord dans un sens pendant un nombre déterminé de cavités puis dans l'autre sens pendant un autre nombre déterminé de cavités. Le nombre de cavités est calculé par le microprocesseur sur la base des informations reçues par un détecteur 41 placé au niveau des cavités 40 de la roue. Les nombres déterminés de cavités correspondent aux positions extrêmes de l'élément mobile 29.

Avantageusement, la roue pleine 39 comprend un autre évidement 42 sous forme d'un demi-cercle concentrique à la roue 39 qui permet de déterminer la position de l'élément mobile 29. Dans la position neutre ou initiale de l'élément mobile 29, la roue 39 est disposée de sorte que le détecteur 41 détecte l'interface entre l'évidement 42 semi-circulaire et le plein.

Dans un exemple particulier, le dispositif comporte un moyen pour détecter la présence d'un tube souple dans l'ensemble de réception. Notamment, un détecteur 43 de présence d'un tube souple dans au moins un des logements est prévu. Lorsque le tube souple 21 est inséré dans un des logements prévus dans l'ensemble de réception 26, une tige mobile 44 agencée dans ledit logement vient pousser une languette 45 d'un contacteur d'un détecteur qui émet alors un signal. Ce détecteur 43 de présence lié à l'unité de commande empêche avantageusement le fonctionnement du dispositif lorsque aucun tube n'est disposé dans l'ensemble de réception.

Dans un autre exemple, le dispositif de rupture comprend un moyen de détermination de l'énergie utilisée pour la rupture de la zone de fragilité 24, par exemple par mesure de l'énergie utilisée par l'organe d'entraînement 36. Le courant utilisé par le moteur pour déplacer l'élément mobile est proportionnel à la force nécessaire pour effectuer le mouvement. Ce courant est mesuré afin de détecter la rupture de l'élément de fermeture. Ainsi, il est prévu que l'élément mobile du dispositif de rupture poursuive son mouvement de va et vient jusqu'à la détection d'une chute du courant révélant la rupture complète de l'élément de fermeture.

Tous les composants du dispositif, y compris l'ensemble de réception, l'organe d'entraînement, l'unité de commande sont organisés dans un boîtier rigide.

Différentes formes sont envisagées pour ce dispositif. Une première forme consiste à intégrer ce dispositif dans un outil portable. Le dispositif de rupture comprend alors une poignée. Notamment, le dispositif de rupture est utilisé lors de l'étape de filtration du sang ou d'un de ses composants, lorsque le système à poches est suspendu à un portique, pour amorcer la filtration. Sur la figure 1, ce dispositif est utilisé lorsque la poche de recueil 2 est suspendue à un portique pour ouvrir le deuxième élément de fermeture 17.

Une deuxième forme est un module autonome dans lequel est intégré ce dispositif. L'utilisateur vient alors disposer les éléments de fermetures dans ce dispositif selon leur besoin.

Une troisième forme est un module directement intégré dans un appareil de traitement d'un fluide biologique tel que le sang. En effet, il est classique que la centrifugation et/ou la séparation soit réalisées par des appareils dédiés, telles que des séparateurs, des centrifugeuses ou des presses telles que celles décrites dans le document WO 2005/002644.

L'intégration de ce dispositif de rupture dans les appareils de traitement du sang facilite l'utilisation de ces appareils. L'unité de commande est programmée pour réaliser la rupture des éléments de fermeture selon un processus déterminé par avance.

Selon un deuxième aspect de l'invention, on décrit maintenant un procédé pour rompre un élément de fermeture 20 à l'aide d'un dispositif selon le premier aspect. Le procédé comprend les étapes de :
- placer le tube souple 21 comportant l'élément de fermeture 20 dans l'ensemble de réception dudit dispositif avec la zone de fragilité 24 disposée entre l'élément fixe 27 et l'élément mobile 29,
- actionner l'organe 36 pour entraîner en déplacement l'élément mobile 29 d'un côté puis de l'autre de l'axe médian 31 de sorte à provoquer la rupture de la zone de fragilité 24 de l'élément de fermeture 20.

Avantageusement, la première partie 22 de l'élément de fermeture est logée dans le logement 28 de l'élément fixe 27 et la deuxième partie 23 de l'élément de fermeture est logée dans le logement 30 de l'élément mobile 29.

Pour casser la zone de fragilité, un ou plusieurs mouvements de l'élément mobile 29 de part et d'autre de l'axe médian 31 sont réalisés.

## Revendications

1. Dispositif destiné à rompre au moins un élément de fermeture (20) disposé à l'intérieur d'un tube souple (21), ledit élément de fermeture (20) comportant une première partie (22) et une deuxième partie (23) séparées par une zone de fragilité (24), ladite zone de fragilité (24) pouvant être rompue pour permettre l'écoulement de fluide à l'intérieur dudit tube souple (21), ledit dispositif comprenant un ensemble de réception (26) dudit tube souple (21), ledit ensemble (26) comprenant un élément fixe (27) pourvu d'un premier logement (28) destiné à recevoir une première portion dudit tube souple, et un élément mobile (29) pourvu d'un deuxième logement (30) destiné à recevoir une deuxième portion dudit tube souple, les deux logements (28,30) étant alignés suivant un axe médian (31) définissant une position neutre de l'élément mobile (29), ledit dispositif étant **caractérisé en ce qu'**il comprend un organe (36) d'entraînement en déplacement de l'élément mobile (29) de part et d'autre de sa position neutre, de sorte à pouvoir provoquer la rupture de la zone de fragilité (24) de l'élément de fermeture (20) lorsque le tube souple (21) est placé dans l'ensemble de réception (26).

2. Dispositif selon la revendication 1, **caractérisé en ce qu'**il comprend un autre élément fixe (27) et un autre élément mobile (29), l'organe (36) étant agencé pour entraîner en déplacement les organes mobiles (29) de sorte à provoquer la rupture simultanée de la zone de fragilité (24) de deux éléments de fermeture (20).

3. Dispositif selon la revendication 1 ou 2, **caractérisé en ce que** l'élément mobile (29) est entraîné en déplacement selon un mouvement linéaire.

4. Dispositif selon la revendications 1 ou 2, **caractérisé en ce que** l'élément mobile (29) est entraîné en déplacement selon un mouvement de rotation.

5. Dispositif selon la revendication 4, **caractérisé en ce que** le mouvement de rotation est un mouvement d'au moins 30° de part et d'autre de l'axe médian (31).

6. Dispositif selon la revendication 5, **caractérisé en ce que** l'axe de rotation de l'élément mobile passe par l'axe médian (31).

7. Dispositif selon l'une des revendications 1 à 6, **caractérisé en ce qu'**au moins un des logements (28) est formé à l'intérieur d'une gorge en forme de U.

8. Dispositif selon l'une des revendications 1 à 7, **caractérisé en ce qu'**au moins un des logements (28) comprend une pince (33) destinée à serrer une portion du tube souple (21).

9. Dispositif selon l'une des revendications 1 à 8, **caractérisé en ce qu'**il comporte un moyen pour détecter la présence d'un tube souple (21) dans l'ensemble de réception (26).

10. Dispositif selon l'une quelconque des revendications 1 à 9, **caractérisé en ce qu'**il comporte un moyen de détermination de l'énergie utilisée pour la rupture de la zone de fragilité (24).

11. Dispositif selon l'une des revendications 1 à 10, **caractérisé en ce qu'**il comporte une poignée.

12. Appareil de traitement d'un fluide biologique, **caractérisé en ce qu'**il comprend un dispositif selon l'une des revendications 1 à 11.

13. Procédé pour rompre un élément de fermeture à l'aide d'un dispositif selon l'une des revendications 1 à 11, **caractérisé en ce qu'**il comprend les étapes de :
- placer le tube souple (21) comportant l'élément de fermeture (20) dans l'ensemble de réception (26) dudit dispositif avec la zone de fragilité (24) disposée entre l'élément fixe (27) et l'élément mobile (29),
- actionner l'organe (36) pour entraîner en déplacement l'élément mobile (29) de part et d'autre de sa position neutre de sorte à provoquer la rupture de la zone de fragilité (24) de l'élément de fermeture (20).

## Patentansprüche

1. Vorrichtung, die dazu bestimmt ist, mindestens ein Verschlusselement (20) zu brechen, das im Inneren eines flexiblen Rohres (21) angeordnet ist, wobei das Verschlusselement (20) einen ersten Abschnitt (22) und einen zweiten Abschnitt (23) aufweist, die durch eine Brüchigkeitszone (24) getrennt sind, wobei die Brüchigkeitszone (24) gebrochen werden kann, um die Fluidströmung im Inneren des flexiblen Rohres (21) zu erlauben, wobei die Vorrichtung eine Einheit zum Aufnehmen (26) des flexiblen Rohres (21) aufweist, wobei die Einheit (26) ein feststehendes Element (27) umfasst, das mit einer ersten Aufnahme (28) versehen ist, die dazu bestimmt ist, einen ersten Abschnitt des flexiblen Rohres aufzunehmen, und ein bewegliches Element (29), das mit einer zweiten Aufnahme (30) versehen ist, die dazu bestimmt ist, einen zweiten Abschnitt des flexiblen Rohres aufzunehmen, wobei die beiden Aufnahmen (28, 30) entlang einer Mittelachse (31) ausgerichtet sind, die eine neutrale Position des beweglichen Elements (29) definiert, wobei die Vorrichtung **dadurch gekennzeichnet ist, dass** sie ein Antriebsorgan (36) zum Verschieben des beweglichen Elements (29) beiderseits seiner neutralen Position umfasst, um den Bruch der Brüchigkeitszone (24) des Verschlusselements (20) bewirken zu können, wenn das flexible Rohr (21) in der Einheit zum Aufnehmen (26) platziert wird.

2. Vorrichtung nach Anspruch 1, **dadurch gekennzeichnet, dass** sie ein weiteres feststehendes Element (27) und ein weiteres bewegliches Element (29) umfasst, wobei das Organ (36) angeordnet ist, um die beweglichen Organe (29) so zum Verschieben anzutreiben, dass sie den gleichzeitigen Bruch der Brüchigkeitszone (24) von zwei Verschlusselementen (20) bewirken.

3. Vorrichtung nach Anspruch 1 oder 2, **dadurch gekennzeichnet, dass** das bewegliche Element (29) zum Verschieben entsprechend einer linearen Bewegung angetrieben wird.

4. Vorrichtung nach Anspruch 1 oder 2, **dadurch gekennzeichnet, dass** das bewegliche Element (29) zum Verschieben entsprechend einer Rotationsbewegung angetrieben wird.

5. Vorrichtung nach Anspruch 4, **dadurch gekennzeichnet, dass** die Rotationsbewegung eine Bewegung um mindestens 30° beiderseits der Mittelachse (31) ist.

6. Vorrichtung nach Anspruch 5, **dadurch gekennzeichnet, dass** die Rotationsachse des beweglichen Elements durch die Mittelachse (31) führt.

7. Vorrichtung nach einem der Ansprüche 1 bis 6, **dadurch gekennzeichnet, dass** mindestens eine der Aufnahmen (28) im Inneren einer Nut in Form eines U gebildet wird.

8. Vorrichtung nach einem der Ansprüche 1 bis 7, **dadurch gekennzeichnet, dass** mindestens eine der Aufnahmen (28) einen Greifer (33) umfasst, der dazu bestimmt ist, einen Abschnitt des flexiblen Rohrs (21) zu klemmen.

9. Vorrichtung nach einem der Ansprüche 1 bis 8, **dadurch gekennzeichnet, dass** sie ein Mittel aufweist, um das Vorhandensein eines flexiblen Rohres (21) in der Einheit zum Aufnehmen (26) zu detektieren.

10. Vorrichtung nach einem der Ansprüche 1 bis 9, **dadurch gekennzeichnet, dass** sie ein Mittel zum Bestimmen der für den Bruch der Brüchigkeitszone (24) verwendeten Energie aufweist.

11. Vorrichtung nach einem der Ansprüche 1 bis 10, **dadurch gekennzeichnet, dass** sie einen Griff aufweist.

12. Gerät zum Behandeln eines biologischen Fluids, **dadurch gekennzeichnet, dass** es eine Vorrichtung nach einem der Ansprüche 1 bis 11 umfasst.

13. Verfahren zum Brechen eines Verschlusselements mithilfe einer Vorrichtung nach einem der Ansprüche 1 bis 11, **dadurch gekennzeichnet, dass** es die folgenden Schritte umfasst:
- Platzieren des flexiblen Rohrs (21), das ein Verschlusselement (20) aufweist, in die Einheit zum Aufnehmen (26) der Vorrichtung, wobei die Brüchigkeitszone (24) zwischen dem feststehenden Element (27) und dem beweglichen Element (29) angeordnet ist,
- Betätigen des Organs (36), um das bewegliche Element (29) beiderseits seiner neutralen Position zum Verschieben anzutreiben, um den Bruch der Brüchigkeitszone (24) des Verschlusselements (20) zu bewirken.

## Claims

1. Device intended to break at least one closure element (20) arranged inside a flexible tube (21), said closure element (20) comprising a first part (22) and a second part (23) separated by a weakening zone (24), said weakening zone (24) able to be broken in order to enable fluid to flow inside said flexible tube (21), said device comprising an assembly for holding (26) said flexible tube (21), said assembly (26) comprising a fixed element (27) provided with a first housing (28) intended to hold a first portion of said flexible tube, and a mobile element (29) provided with a second housing (30) intended to hold a second portion of said flexible tube, the two housings (28,30) being aligned along a centre line (31) defining a neutral position of the mobile element (29), said device being **characterised in that** it comprises a member (36) for moving the mobile element (29) to either side of its neutral position, in such a way as to be able to break the weakening zone (24) of the closure element (20) when the flexible tube (21) is placed in the assembly for holding (26).

2. Device according to claim 1, **characterised in that** it comprises another fixed element (27) and another mobile element (29), the member (36) being arranged to move the mobile members (29) in such a way as to simultaneous break the weakening zone (24) of two closure elements (20) .

3. Device according to claim 1 or 2, **characterised in that** the mobile element (29) is driven in displacement according to a linear movement.

4. Device according to claims 1 or 2, **characterised in that** the mobile element (29) is driven in displacement according to a rotational movement.

5. Device according to claim 4, **characterised in that** the rotational movement is a movement of at least 30° on either side of the centre line (31).

6. Device according to claim 5, **characterised in that** the axis of rotation of the mobile element passes through the centre line (31).

7. Device according to one of claims 1 to 6, **characterised in that** at least one of the housings (28) is formed inside a U-shaped groove.

8. Device according to one of claims 1 to 7, **characterised in that** at least one of the housings (28) comprises a clamp (33) intended to tighten a portion of the flexible tube (21).

9. Device according to one of claims 1 to 8, **characterised in that** it comprises a means for detecting the presence of a flexible tube (21) in the assembly for holding (26).

10. Device according to any of claims 1 to 9, **characterised in that** it comprises a means for determining the energy used for the breaking of the weakening zone (24).

11. Device according to one of claims 1 to 10, **characterised in that** it comprises a handle.

12. Apparatus for treating a biological fluid, **characterised in that** it comprises a device according to one of claims 1 to 11.

13. Method for breaking a closure element using a device according to one of claims 1 to 11, **characterised in that** it comprises the steps of:
- placing the flexible tube (21) comprising the closure element (20) in the assembly for holding (26) said device with the weakening zone (24) arranged between the fixed element (27) and the mobile element (29),
- actuating the member (36) for moving the mobile element (29) to either side of its neutral position in such a way as to break the weakening zone (24) of the closure element (20).
